# EUROPEAN PATENT APPLICATION

(11) **EP 4 516 795 A1**
(43) Date of publication of application: **05.03.2025**
(21) Application number: 23795377.3
(22) Date of filing: 25.04.2023
(51) Int. Cl.: C07D 519/00, A61K 31/4985, A61P 35/00

(54) **CRYSTAL FORM OF FGFR4 INHIBITOR AND USE THEREOF**

(30) Priority: 26.04.2022 CN 202210443921
(71) Applicant: CSPC Zhongqi Pharmaceutical Technology (Shijiazhuang) Co., Ltd., Shijiazhuang, Hebei 050035 (CN); Hangzhou Innogate Pharma Co., Ltd., Hangzhou, Zhejiang 311121 (CN); CSPC OUYI Pharmaceutical co., Ltd., Shijiazhuang, Hebei 052165 (CN)
(72) Inventor: PENG, Xiaoshi, Shijiazhuang, Hebei 050035 (CN); ZHANG, Hancheng, Hangzhou, Zhejiang 311121 (CN); YANG, Min, Shijiazhuang, Hebei 050035 (CN); YE, Xiangyang, Hangzhou, Zhejiang 311121 (CN); ZHANG, Dandan, Shijiazhuang, Hebei 050035 (CN); LIANG, Xinlei, Shijiazhuang, Hebei 050035 (CN); YAN, Cuiqin, Shijiazhuang, Hebei 050035 (CN); LI, Yuqin, Shijiazhuang, Hebei 050035 (CN); YAO, Lei, Shijiazhuang, Hebei 050035 (CN)
(74) Representative: Gille Hrabal Partnerschaftsgesellschaft mbB Patentanwälte
(86) International application number: PCT/CN2023/090492
(87) International publication number: WO 2023/207944

(57) **Abstract**

The present invention relates to a crystalline form of an FGFR4 inhibitor and use thereof. The present invention provides a compound of formula B in crystalline form. The compound of formula B of the present invention has excellent FGFR4 kinase inhibitory activity and Huh-7 tumor cell proliferation inhibition. The pharmacokinetic effect of the present compound is better than that of its enantiomers or its racemates. The crystalline form of the present compound and especially the optional crystalline forms thereof have good solubility, stable quality, and thermodynamic stability, and can be easily made into a medicament.

## Description

### TECHNICAL FIELD

The present disclosure belongs to the field of pharmaceutical chemistry, and particularly relates to a crystalline form of an FGFR4 selective inhibitor and use thereof.

### BACKGROUND

FGFR (fibroblast growth factor receptor) is a receptor protein tyrosine kinase, and has 4 receptors FGFR1, FGFR2, FGFR3, and FGFR4, which play a key role in maintaining the growth, proliferation, apoptosis, migration, etc. of cells. FGFR activating mutations can not only promote the proliferation of malignant tumor cells and inhibit apoptosis, but also play an important role in the processes of tumor angiogenesis, tumor invasion, tumor metastasis, and the like. The high expression of FGFR exists in non-small cell lung cancer, liver cancer, breast cancer, bladder cancer, and other cancers, so that FGFR small molecule kinase inhibitors are a promising treatment method for tumor patients with abnormal expression of FGFR, and the research and development of selective FGFR small molecule inhibitors are receiving more and more attention.

Liver cancer is one of the most malignant tumors with the highest morbidity and mortality, and there are 466,000 new cases of liver cancer and 422,000 cases of liver cancer deaths in China each year. Studies have shown that the FGFR4-FGF19 signaling system is closely related to hepatocellular carcinomas (HCCs), and FGFR4 is an FGFR subtype highly expressed in human hepatocytes, with various FGFR4 variations found in patients with liver cancer. Selective inhibition of FGFR4 without inhibition of other subtypes FGFR1, FGFR2, and FGFR3 may avoid certain toxicity, so that FGFR4 is likely to become an important target for treating liver cancer. Clinical studies have shown that FGFR inhibitors may be used in the treatment of various cancers, but there is an urgent need to develop selective FGFR4 inhibitors for the treatment of various tumors, particularly for the treatment of liver cancer.

The Chinese Patent Application CN108948004A discloses an FGFR4 inhibitor represented by formula (I) shown below: 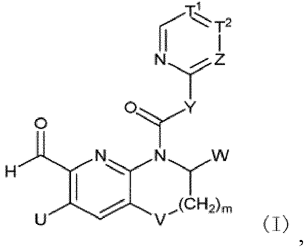 wherein Example 5 discloses a compound with a structure shown below:

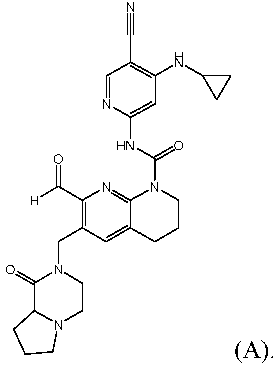

### SUMMARY

Compound of formula A has two isomers: S-isomer (represented by formula B shown below) and R-isomer (represented by formula C shown below). The inventors have found that compound of formula B has better pharmacokinetic properties and higher druggability compared to compound of formula A or compound of formula C. Based on such finding, the inventors systematicly study on the crystalline forms of compound of formula B to identify a stable crystalline form that might be used for subsequent development.

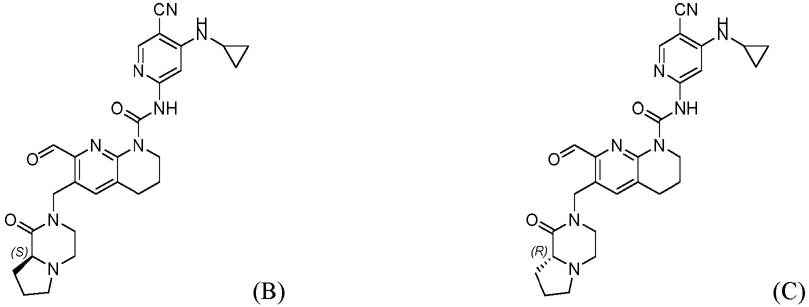

In order to solve the problems described above, in a first aspect, the present disclosure provides a compound of formula B in a crystalline form:

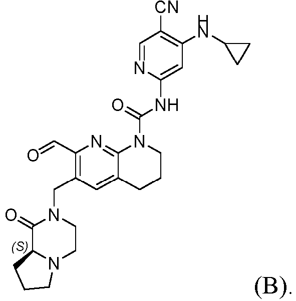

In some embodiments, the compound of formula B in the crystalline form is an anhydrate.

In a second aspect, the present disclosure provides crystalline form I of the compound of formula B, wherein the crystalline form I of the compound of formula B has characteristic peaks at 2θ angles of 7.4±0.2° and 25.0±0.2° by X-ray powder diffraction using Cu-Kα radiation.

In some embodiments, the crystalline form I of the compound of formula B has characteristic peaks at 2θ angles of 7.4±0.2°, 15.9±0.2°, 17.4±0.2°, 20.9±0.2°, and 25.0±0.2° by X-ray powder diffraction using Cu-Kα radiation.

In some embodiments, the crystalline form I of the compound of formula B has characteristic peaks at 2θ angles of 7.4±0.2°, 9.8±0.2°, 15.9±0.2°, 17.4±0.2°, 20.9±0.2°, and 25.0±0.2° by X-ray powder diffraction using Cu-Kα radiation.

In some embodiments, the crystalline form I of the compound of formula B has characteristic peaks at 2θ angles of 7.4±0.2°, 9.8±0.2°, 15.9±0.2°, 17.4±0.2°, 20.9±0.2°, 22.4±0.2°, and 25.0±0.2° by X-ray powder diffraction using Cu-Kα radiation.

In some embodiments, the crystalline form I of the compound of formula B has characteristic peaks at 2θ angles of 7.4±0.2°, 9.8±0.2°, 11.7±0.2°, 12.0±0.2°, 14.6±0.2°, 15.9±0.2°, 17.4±0.2°, 20.9±0.2°, 22.4±0.2°, and 25.0±0.2° by X-ray powder diffraction using Cu-Kα radiation.

In some embodiments, the crystalline form I of the compound of formula B has characteristic peaks at 2θ angles of 7.4±0.2°, 9.8±0.2°, 11.7±0.2°, 12.0±0.2°, 14.6±0.2°, 15.9±0.2°, 17.4±0.2°, 19.7±0.2°, 20.9±0.2°, 22.4±0.2°, 23.6±0.2°, and 25.0±0.2° by X-ray powder diffraction using Cu-Kα radiation.

In some embodiments, the crystalline form I of the compound of formula B has characteristic peaks at 2θ angles of 7.4±0.2°, 9.8±0.2°, 11.7±0.2°, 12.0±0.2°, 14.6±0.2°, 15.5±0.2°, 15.9±0.2°, 17.4±0.2°, 18.8±0.2°, 19.7±0.2°, 20.9±0.2°, 22.0±0.2°, 22.4±0.2°, 23.6±0.2°, 25.0±0.2°, and 27.8±0.2° by X-ray powder diffraction using Cu-Kα radiation.

In some embodiments, the crystalline form I of the compound of formula B has characteristic peaks at 2θ angles of 7.4±0.2°, 9.8±0.2°, 11.7±0.2°, 12.0±0.2°, 14.6±0.2°, 15.5±0.2°, 15.9±0.2°, 17.4±0.2°, 18.5±0.2°, 18.8±0.2°, 19.7±0.2°, 20.9±0.2°, 22.0±0.2°, 22.4±0.2°, 23.6±0.2°, 25.0±0.2°, and 27.8±0.2° by X-ray powder diffraction using Cu-Kα radiation.

In some embodiments, the crystalline form I of the compound of formula B has characteristic peaks at 2θ angles of 7.4±0.2°, 9.8±0.2°, 11.7±0.2°, 12.0±0.2°, 14.6±0.2°, 15.5±0.2°, 15.9±0.2°, 17.4±0.2°, 18.8±0.2°, 19.7±0.2°, 20.9±0.2°, 22.0±0.2°, 22.4±0.2°, 23.6±0.2°, 25.0±0.2°, 26.0±0.2°, and 27.8±0.2° by X-ray powder diffraction using Cu-Kα radiation.

In some embodiments, the crystalline form I of the compound of formula B has characteristic peaks at 2θ angles of 7.4°, 9.8°, 11.7°, 12.0°, 14.6°, 15.5°, 15.9°, 17.4°, 18.5°, 18.8°, 19.6°, 20.9°, 22.0°, 22.4°, 23.6°, 24.8°, and 27.7° by X-ray powder diffraction using Cu-Kα radiation.

In some embodiments, the crystalline form I of the compound of formula B has characteristic peaks at 2θ angles of 7.5°, 9.9°, 11.8°, 12.1°, 14.7°, 15.6°, 16.1°, 17.6°, 19.0°, 19.9°, 21.1°, 22.2°, 22.6°, 23.8°, 25.2°, 26.0°, and 28.0° by X-ray powder diffraction using Cu-Kα radiation.

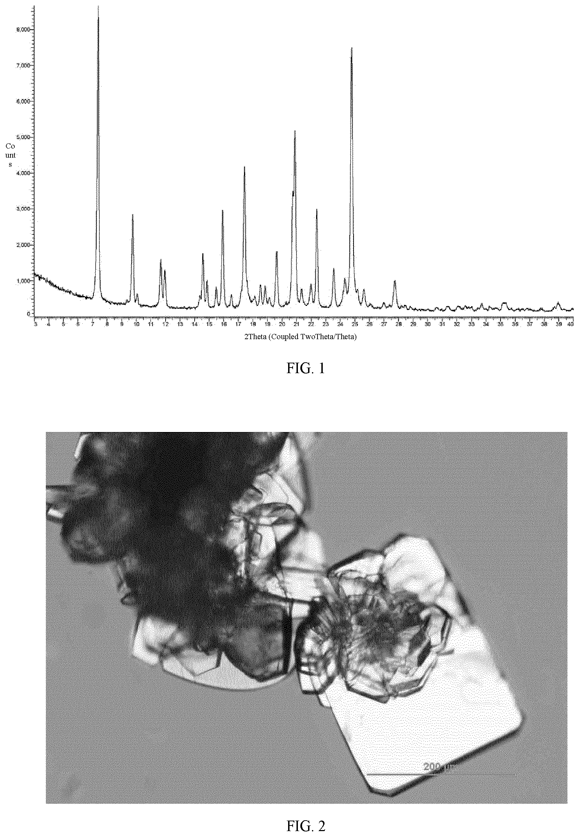

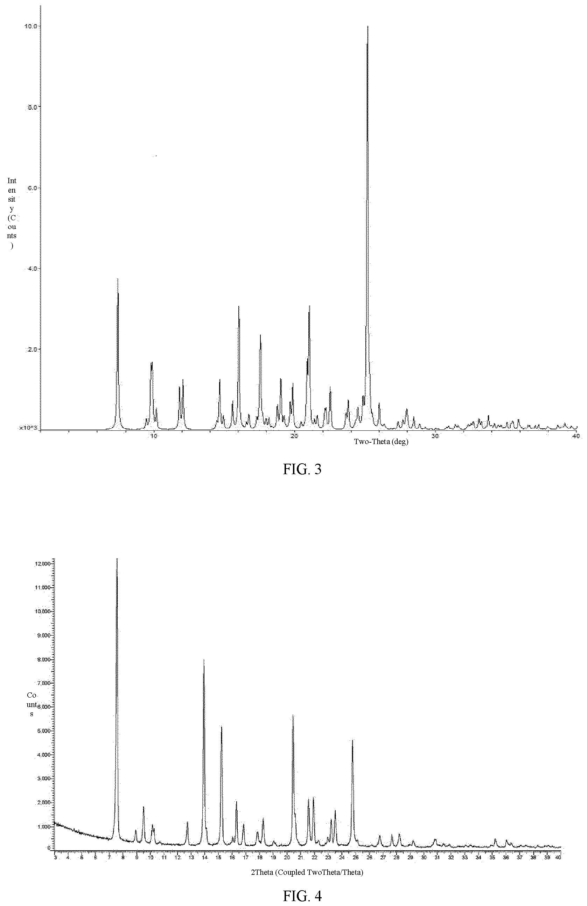

In some embodiments, the crystalline form I of the compound of formula B has an X-ray powder diffraction pattern (XRPD) substantially as shown in FIG. 1 or FIG. 3 by using Cu-Kα radiation.

In some embodiments, the crystalline form I of the compound of formula B, whose single crystal is examined using CuKα radiation and belongs to the monoclinic crystal system with a *P*2₁ space group, and the unit cell parameters are as follows: {a = 10.8374(2) Ǻ, b = 23.6358(3) Ǻ, c = 10.96160(10) Ǻ, α = 90°, β = 117.360(2)°, γ = 90°, V = 2493.73(7) Ǻ³}.

In a third aspect, the present disclosure provides crystalline form II of the compound of formula B, wherein the crystalline form II has characteristic peaks at 2θ angles of 7.6±0.2° and 14.0±0.2° by X-ray powder diffraction using Cu-Kα radiation.

In some embodiments, the crystalline form II of the compound of formula B has characteristic peaks at 2θ angles of 7.6±0.2°, 14.0±0.2°, 15.2±0.2°, 20.4±0.2°, and 24.8±0.2° by X-ray powder diffraction using Cu-Kα radiation.

In some embodiments, the crystalline form II of the compound of formula B has characteristic peaks at 2θ angles of 7.6±0.2°, 9.5±0.2°, 14.0±0.2°, 15.2±0.2°, 16.3±0.2°, 20.4±0.2°, 21.6±0.2°, 21.9±0.2°, 23.2±0.2°, 23.5±0.2°, and 24.8±0.2° by X-ray powder diffraction using Cu-Kα radiation. In some embodiments, the crystalline form II of the compound of formula B has characteristic peaks at 2θ angles of 7.6±0.2°, 9.5±0.2°, 10.2±0.2°, 12.7±0.2°, 14.0±0.2°, 15.2±0.2°, 16.3±0.2°, 16.8±0.2°, 18.3±0.2°, 20.4±0.2°, 21.6±0.2°, 21.9±0.2°, 23.2±0.2°, 23.5±0.2°, and 24.8±0.2° by X-ray powder diffraction using Cu-Kα radiation.

In some embodiments, the crystalline form II of the compound of formula B has characteristic peaks at 2θ angles of 7.6±0.2°, 9.5±0.2°, 10.2±0.2°, 12.7±0.2°, 14.0±0.2°, 15.2±0.2°, 16.3±0.2°, 16.8±0.2°, 17.9±0.2°, 18.3±0.2°, 20.4±0.2°, 21.6±0.2°, 21.9±0.2°, 23.2±0.2°, 23.5±0.2°, 24.8±0.2°, 26.8±0.2°, 27.7±0.2°, and 28.2±0.2° by X-ray powder diffraction using Cu-Kα radiation.

In some embodiments, the crystalline form II of the compound of formula B has an X-ray powder diffraction pattern (XRPD) substantially as shown in FIG. 4 by using Cu-Kα radiation.

In a fourth aspect, the present disclosure provides crystalline form III of the compound of formula B, wherein the crystalline form III has characteristic peaks at a 2θ angle of 4.4±0.2° by X-ray powder diffraction using Cu-Kα radiation.

In some embodiments, the crystalline form III of the compound of formula B has characteristic peaks at 2θ angles of 4.4±0.2°, 10.4±0.2°, 13.4±0.2°, 25.3±0.2°, and 25.8±0.2° by X-ray powder diffraction using Cu-Kα radiation.

In some embodiments, the crystalline form III of the compound of formula B has characteristic peaks at 2θ angles of 4.4±0.2°, 10.4±0.2°, 13.4±0.2°, 14.0±0.2°, 18.1±0.2°, 18.5±0.2°, 20.5±0.2°, 24.3±0.2°, 25.3±0.2°, and 25.8±0.2° by X-ray powder diffraction using Cu-Kα radiation.

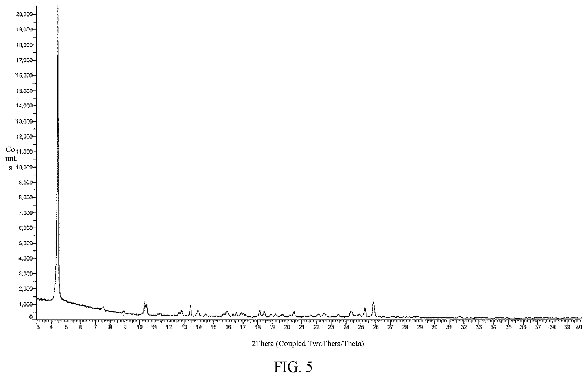

In some embodiments, the crystalline form III of the compound of formula B has an X-ray powder diffraction pattern (XRPD) substantially as shown in FIG. 5 by using Cu-Kα radiation.

In a fifth aspect, the present disclosure further provides a pharmaceutical composition, which comprises the compound of formula B in the crystalline form described above, or one or a mixture of more of the crystalline form I, the crystalline form II, and the crystalline form III of the compound of formula B described above.

In some embodiments, the pharmaceutical composition comprises the compound of formula B in the crystalline form, or one or a mixture of more of the crystalline form I, the crystalline form II, and the crystalline form III of the compound of formula B described above, and one or more pharmaceutically acceptable carriers.

In a sixth aspect, the present disclosure further provides use of the compound of formula B in the crystalline form described above, or the crystalline form I, the crystalline form II, and the crystalline form III of the compound of formula B described above, or the pharmaceutical composition described above in the manufacture of a medicament for treating a disease associated with FGFR4 activity or expression.

In some embodiments, the present disclosure further provides the compound of formula B in the crystalline form described above, or the crystalline form I, the crystalline form II, and the crystalline form III of the compound of formula B described above, or the pharmaceutical composition described above for use in treating a disease associated with FGFR4 activity or expression.

In some embodiments, the present disclosure further provides a method for treating a disease associated with FGFR4 activity or expression, which comprises administering to a subject in need thereof an effective amount of the compound of formula B in the crystalline form described above, or the crystalline form I, the crystalline form II, and the crystalline form III of the compound of formula B described above, or the pharmaceutical composition described above.

In some embodiments, the disease associated with the FGFR4 activity or expression is selected from cancers, e.g., lung cancer, bladder cancer, breast cancer, gastric cancer, liver cancer, salivary gland sarcoma, ovarian cancer, prostate cancer, cervical cancer, epithelial cell carcinoma, multiple myeloma, pancreatic cancer, lymphoma, chronic myelogenous leukemia, lymphocytic leukemia, cutaneous T-cell lymphoma, etc.; the lung cancer is preferably non-small cell lung cancer.

In some embodiments, the disease associated with the FGFR4 activity or expression is selected from bone-related diseases, e.g., osteogenesis imperfecta, achondroplasia, dwarfism, Crouzon syndrome, etc.

In some embodiments, the disease associated with the FGFR4 activity or expression is selected from T cell-regulated inflammation and autoimmune diseases, e.g., rheumatoid arthritis, collagen II arthritis, multiple sclerosis, systemic lupus erythematosus, psoriasis, juvenile onset diabetes, Sjogren's syndrome, thyroid diseases, sarcoidosis, inflammatory bowel disease, celiac disease, etc. The medicament is administered to a "subject" or "patient" in an effective dose. The "subject" and "patient" include all members of the animal kingdom, including but not limited to, mammals (e.g., mice, rats, cats, monkeys, dogs, horses, pigs, etc.) and humans.

### Definitions and Description

Unless otherwise stated, the following terms and phrases used herein are intended to have the following meanings. A particular phrase or term, unless otherwise specifically defined, should not be considered as uncertain or unclear, but construed according to its common meaning.

The compound of formula B in the crystalline form mentioned herein includes an anhydrous and solvent-free form, a hydrate form, a solvate form, and a cocrystalline form of the compound of formula B.

In the X-ray powder diffraction pattern, the term "substantially" or "substantially as shown in the figure" means that for a certain crystalline form that is substantially pure, at least 50%, or at least 60%, or at least 70%, or at least 80%, or at least 90%, or at least 95%, or at least 96%, or at least 97%, or at least 98%, or at least 99% of the peaks in the X-ray powder diffraction pattern appear in the given pattern. Further, when the content of a certain crystalline form in the product is gradually reduced, some diffraction peaks attributed to the crystalline form in the X-ray powder diffraction pattern may be reduced due to the detection sensitivity of the instrument. In addition, there may be slight errors in the peak positions for any given crystalline form, as is also well known in the field of crystallography. For example, the peak positions may shift due to temperature changes, sample movement, or calibration of the instrument, etc. when analyzing a sample, and the error in the measurement of 2θ value is sometimes about ±0.3°, and usually about ±0.2°. Therefore, this error should be taken into account when determining the structure of each crystalline form. The terms "substantially" or "substantially as shown in the figure" are also intended to encompass such errors in diffraction peak positions, meaning ±0.3°, preferably ±0.2°.

The term "tumor" includes benign tumors, malignant tumors, and borderline tumors, wherein malignant tumors are also collectively referred to as cancers.

The term "preventing" as used herein refers to a compound or drug that, when used for treating a disease or condition (e.g., cancer), can reduce the frequency of or delay the onset of symptoms of a medical condition in a subject as compared with a subject who has not been administered the compound or drug (e.g., a combination product as claimed herein).

The term "treating" as used herein refers to alleviating, relieving, or ameliorating a symptom of a disease or condition, ameliorating an underlying metabolic-induced symptom, and inhibiting a disease or symptom, e.g., arresting the progression of a disease or condition, relieving a disease or condition, causing regression of a disease or condition, relieving a disorder caused by a disease or condition, or arresting a symptom of a disease or condition.

The term "pharmaceutically acceptable carrier" or "pharmaceutically acceptable auxiliary material" refers to those carriers or auxiliary materials that do not have a significant irritating effect on organisms and do not impair the bioactivity and performance of the active compound.

All solvents used in the present application are commercially available and could be used without further purification.

### Beneficial effects:

The present disclosure provides a compound of formula B in a crystalline form. The inventors surprisingly found that the compound of formula B has a better pharmacokinetic effect compared to an enantiomer or a racemate thereof, and the crystalline form of formula B, particularly the preferred crystalline form of formula B, has stable quality, stable thermodynamics, and desirable druggability.

### BRIEF DESCRIPTION OF THE DRAWINGS

FIG. 1 shows an XRPD pattern of crystalline form I of the compound of formula B.
FIG. 2 is a single crystal PLM image of the crystalline form I of the compound of formula B.
FIG. 3 shows a single crystal calculated XRPD pattern of the crystalline form I of the compound of formula B.
FIG. 4 shows an XRPD pattern of crystalline form II of the compound of formula B.
FIG. 5 shows an XRPD pattern of crystalline form III of the compound of formula B.

### DETAILED DESCRIPTION

The technical solutions of the present disclosure will be further described in detail with reference to the following specific examples. The following examples are merely exemplary illustration and explanation of the present disclosure, and should not be construed as limiting the protection scope of the present disclosure. All techniques implemented based on the content described above of the present disclosure are encompassed within the protection scope of the present disclosure.

Unless otherwise stated, the starting materials and reagents used in the following examples are all commercially available products or can be prepared by using known methods.

In the following examples, the detection conditions of the patterns are as follows:
1. XRPD detection conditions
Instrument: BRUKER D8 Advance X-ray powder diffractometer (BRUKER GER), Germany.
Conditions: Cu-Kα radiation; tube voltage: 40 kV; tube current: 40 mA; 2θ scanning range: 3-40°; scanning step size: 0.02°; scanning speed: 0.2 s/step; sample tray: a zero background sample tray.

2. Dynamic vapor sorption/desorption (DVS) analysis
Instrument: DVS Intrinsic plus (SMS, UK).
Method: a sample is placed in a tared sample basket, the weight of the sample is automatically measured, and the sample is dried at 40 °C/0% RH until dm/dt is less than 0.002% and then cooled to 25 °C. The instrument parameters are shown in Table 1.

**Table 1. Parameters of dynamic vapor sorption/desorption (DVS) analysis**

| | |
|---|---|
| Duration for each step (min) | 60 min |
| Sample temperature | 25°C |
| Measurement cycle | Full cycle |
| Sorption process | 0, 10, 20, 30, 40, 50, 60, 70, 80, 90 |
| Desorption process | 80, 70, 60, 50, 40, 30, 20, 10, 0 |
| Data storage rate | 5 s |
| Total gas flow | 200 sccm |
| Post-run flow rate | 200 sccm |

3. Single crystal X-ray diffraction analysis
Instrument: Rigaku XtaLAB Synergy-DW diffractometer
Method: single crystal X-ray data are collected at 180 K with Cu Kα radiation (*λ* = 1.54184 Å) as the light source. Data reduction and empirical absorption correction are performed using the CrysAlisPro program, and the structure is analyzed by using the dual space algorithm with the SHELXT program. The coordinates of all non-hydrogen atoms are determined according to a difference Fourier map, and hydrogen atoms are theoretically hydrogenated by being relatively fixed to their parent atoms. The final structure is refined using the SHELXL program based on the *F*² full matrix least square method.

4. Polarizing microscope (PLM) analysis
Instrument: ECLIPSE LV100POL polarizing microscope (Nikon, Japan)
Method: a small amount of sample is placed on a glass slide, and the glass slide is covered after immersion oil is added dropwise to slightly disperse the sample evenly. Microscopic observation and photographing are performed with a 4-20 × objective lens and polarized light.

### Preparation Example 1: Preparation of Compound of Formula B

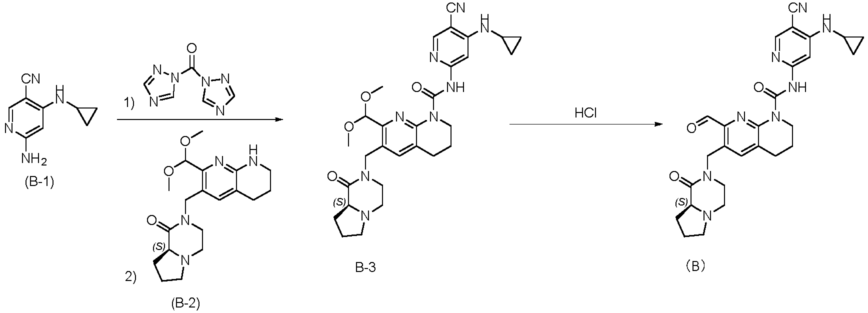

Compound B-1 (6 g, 34.5 mmol) and N,N-carbonyldi(1,2,4-triazole) (17 g, 103.4 mmol) were dissolved in dry N,N-dimethylformamide (60 mL), and the mixture was stirred at room temperature for 2 h. Compound B-2 (8.7 g, 24.1 mmol, prepared using S configuration 10a with reference to Example 10 in WO2017202390A1) was then added, and the reaction system was stirred at room temperature overnight. After completion of the reaction as monitored by HPLC, the reaction liquid was quenched with a 10% lithium chloride solution and extracted three times with ethyl acetate. The organic layers were combined, washed with saturated brine, dried over anhydrous sodium sulfate, and concentrated to obtain a crude product, which was subjected to column chromatography (dichloromethane/methanol = 25/1-20/1) to obtain compound B-3 (1.5 g, 11% yield) as a white solid.

Compound B-3 (1.5 g, 2.7 mmol) was dissolved in tetrahydrofuran (30 mL), and HCl (2 M, aqueous solution) (20 mL) was added dropwise to the solution at room temperature. The reaction liquid was stirred at room temperature for 3 h. The reaction liquid was poured into an appropriate amount of saturated sodium bicarbonate solution, and the resulting mixture was extracted three times with dichloromethane. The organic layers were combined, washed with saturated brine, dried over anhydrous sodium sulfate, and concentrated to obtain a crude product, which was separated and purified by column chromatography (dichloromethane/methanol = 20/1) to obtain the compound of formula B (0.44 g, 32% yield) as an off-white solid. ¹HNMR (500 MHz, CDCl₃) δ 13.56 (s, 1H), 10.17 (s, 1H), 8.11 (s, 1H), 7.85 (s, 1H), 7.57 (s, 1H), 5.21 (s, 1H), 5.00 (dd, J = 46.1, 15.7 Hz, 2H), 4.07 - 3.96 (m, 2H), 3.45 (ddd, J = 12.3, 9.6, 4.8 Hz, 1H), 3.23 (ddd, J = 16.8, 10.7, 6.3 Hz, 2H), 2.98 (dt, J = 12.2, 4.3 Hz, 1H), 2.91 (ddd, J = 9.5, 7.6, 4.8 Hz, 1H), 2.85 (t, J = 6.3 Hz, 2H), 2.79 - 2.69 (m, 1H), 2.61 - 2.51 (m, 2H), 2.23 - 2.14 (m, 1H), 2.00 - 1.94 (m, 2H), 1.94 - 1.69 (m, 3H), 0.93 - 0.85 (m, 2H), 0.63 - 0.55 (m, 2H). MS 515.20 [M+H]⁺.

The XRPD test showed that the solid was in an amorphous form.

### Preparation Example 2: Preparation of Compound of Formula C

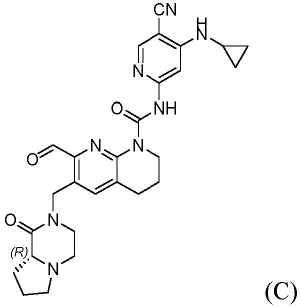

The compound of formula C was obtained by referring to the preparation method in Preparation Example 1 except for replacing B-2 by R configuration. ¹HNMR (500 MHz, CDCl₃) δ 13.56 (s, 1H), 10.17 (s, 1H), 8.11 (s, 1H), 7.85 (s, 1H), 7.57 (s, 1H), 5.21 (s, 1H), 5.00 (dd, J = 47.8, 15.7 Hz, 2H), 4.09 - 3.96 (m, 2H), 3.50 - 3.39 (m, 1H), 3.30 - 3.14 (m, 2H), 3.02 - 2.95 (m, 1H), 2.95 - 2.88 (m, 1H), 2.85 (t, J = 6.2 Hz, 2H), 2.79 - 2.71 (m, 1H), 2.64 - 2.52 (m, 2H), 2.25 - 2.15 (m, 1H), 2.02 - 1.93 (m, 2H), 1.92 - 1.71 (m, 3H), 0.94 - 0.83 (m, 2H), 0.64 - 0.55 (m, 2H). MS 515.16 [M+H]⁺.

### Example 1: Preparation of Crystalline form I of Compound of Formula B

The compound of formula B (20 mg) obtained in Preparation Example 1 was added to a reaction flask, and dimethylsulfoxide (3 mL) was added. The reaction liquid was stirred at 60 °C for 1 h and filtered while hot to obtain a saturated solution. The filtrate obtained by filtration was stirred at room temperature overnight, and the resulting solid was filtered and dried under vacuum at 50 °C for 12 h to obtain the crystalline form I of the compound of formula B.

The obtained crystalline form I exhibited good crystallinity. The XRPD characterization pattern is substantially as shown in FIG. 1, and the characterization data are shown in Table 2. The sample was almost non-hygroscopic, with a hygroscopic weight gain of about 0.24% under the condition of 0-90% RH, and the crystalline form of the sample after the DVS test was not changed.

**Table 2. XRPD characterization data on crystalline form I sample in Example 1**

| 2θ (°) | Relative peak intensity (%) | 2θ (°) | Relative peak intensity (%) |
|---|---|---|---|
| 7.394 | 100.00 | 18.845 | 7.30 |
| 9.759 | 30.20 | 19.636 | 19.30 |
| 11.688 | 16.30 | 20.893 | 63.00 |
| 11.964 | 13.00 | 21.35 | 6.10 |
| 14.582 | 18.90 | 21.987 | 8.00 |
| 14.862 | 8.40 | 22.391 | 35.90 |
| 15.492 | 7.10 | 23.557 | 14.10 |
| 15.936 | 34.70 | 24.784 | 93.30 |
| 17.433 | 48.60 | 25.615 | 6.50 |
| 18.527 | 7.40 | 27.745 | 9.80 |

### Example 2: Preparation of Crystalline form I of Compound of Formula B

The compound of formula B (about 5 mg) obtained in Preparation Example 1 was dissolved in 1 mL of acetonitrile/dichloromethane (V:V = 1:1) at 50 °C. After filtration, a minimal amount of hydroxypropyl methylcellulose (HPMC) was added to the filtrate as a template, and the sample bottle was capped with a sealing film on which holes were made, and placed in a fume hood for slow volatilization at room temperature. After one day, a flaky single crystal was obtained, as shown in FIG. 2. The single crystal sample was used for single crystal X-ray diffraction analysis. The single crystal structure belongs to a *P*2₁ space group of a monoclinic crystal system, and the molecular formula is C₂₇H₃₀N₈O₃. There are two compound molecules of formula B in each asymmetric unit and two asymmetric units in each unit cell. The unit cell parameters are as follows: {a = 10.8374(2) Ǻ, b = 23.6358(3) Ǻ, c = 10.96160(10) Ǻ, α = 90°, β = 117.360(2)°, γ = 90°, V = 2493.73(7) Ǻ³}.

The XRPD pattern calculated from the single crystal structure is shown in FIG. 3, and the results are shown in Table 3, which are substantially consistent with the diffraction peak positions of the XRPD pattern of the crystalline form I obtained in Example 1.

**Table 3. XRPD characterization data calculated from single crystal**

| 2θ (°) | Relative peak intensity (%) | 2θ (°) | Relative peak intensity (%) |
|---|---|---|---|
| 7.478 | 37.7 | 19.882 | 11 |
| 9.9 | 16.7 | 21.059 | 30.4 |
| 11.843 | 10.7 | 22.239 | 5.4 |
| 12.097 | 12.4 | 22.557 | 10.6 |
| 14.719 | 12.4 | 23.822 | 7.4 |
| 15.623 | 6.7 | 25.179 | 100 |
| 16.078 | 30.3 | 26.021 | 6.5 |
| 17.598 | 23.1 | 27.98 | 5.1 |
| 19.043 | 11.9 | | |

### Example 3 to Example 21: Preparation of Crystalline form I of Compound of Formula B

### (1) Cooling crystallization method

Referring to the preparation method in Example 1, the crystalline form I of the compound of formula B was obtained by using the solvent systems in the table below.

**Table 4. Preparation of crystalline form I of compound of formula B by cooling crystallization method**

| Example | Solvent | Result |
|---|---|---|
| 3 | Dichloromethane/acetone (V:V = 2:1) | Crystalline form I |
| 4 | N,N-Dimethylformamide | Crystalline form I |

### (2) Suspension and slurring method

The compound of formula B (20 mg) obtained in Preparation Example 1 was added to a reaction flask, and then different solvents were separately added to prepare suspensions. All suspensions were stirred at room temperature or 50 °C for 3 days, and the resulting solids were filtered and then subjected to XRPD characterization, which showed these solids were the crystalline form I.

**Table 5. Preparation of crystalline form I of compound of formula B by suspension and slurring method**

| **Example** | **Solvent** | **Suspension temperature** | **XRPD result** |
|---|---|---|---|
| 5 | N,N-Dimethylformamide | Room temperature | Crystalline form I |
| 6 | N,N-Dimethylacetamide | Room temperature | Crystalline form I |
| 7 | Acetone/water (V:V = 95:5) | Room temperature | Crystalline form I |
| 8 | Water/methanol (V:V = 5:95) | Room temperature | Crystalline form I |
| 9 | Water/N,N-dimethylformamide (V:V = 5:95) | Room temperature | Crystalline form I |
| 10 | Dichloromethane/methanol (V:V = 1:4) | 50 °C | Crystalline form I |
| 11 | Dichloroethane/ethanol (V:V = 1:4) | 50 °C | Crystalline form I |
| 12 | Dimethylsulfoxide | 50 °C | Crystalline form I |
| 13 | N,N-Dimethylformamide | 50 °C | Crystalline form I |
| 14 | N,N-Dimethylacetamide | 50 °C | Crystalline form I |
| 15 | Dichloromethane/acetone (V:V = 1:4) | 50 °C | Crystalline form I |
| 16 | Acetone/acetonitrile (V:V = 1:4) | 50 °C | Crystalline form I |
| 17 | Acetonitrile/water (V:V = 1:4) | 50 °C | Crystalline form I |
| 18 | Acetone/water (V:V = 95:5) | 50 °C | Crystalline form I |
| 19 | Water/methanol (V:V = 5:95) | 50 °C | Crystalline form I |
| 20 | Water/dimethylsulfoxide (V:V = 5:95) | 50 °C | Crystalline form I |
| 21 | Water/N,N-dimethylformamide (V:V = 5:95) | 50 °C | Crystalline form I |

### Example 22: Preparation of Crystalline form II of Compound of Formula B

The compound of formula B (20 mg) obtained in Preparation Example 1 was added to a reaction flask, dichloromethane (4 mL) was added for dissolution, and acetonitrile (8 mL) was slowly added at room temperature. A large amount of solid precipitated. The resulting solid was subjected to XRPD characterization, which showed the solid was the crystalline form II. The XRPD characterization pattern is substantially as shown in FIG. 4, and the characterization data are shown in Table 6.

**Table 6. XRPD characterization data on crystalline form II sample in Example 22**

| 2θ (°) | Relative peak intensity (%) | 2θ (°) | Relative peak intensity (%) |
|---|---|---|---|
| 7.568 | 100.00 | 20.445 | 53.70 |
| 9.51 | 12.80 | 21.582 | 18.30 |
| 10.152 | 10.80 | 21.936 | 19.60 |
| 12.723 | 8.70 | 23.211 | 11.20 |
| 13.958 | 70.50 | 23.514 | 14.60 |
| 15.244 | 43.40 | 24.773 | 45.90 |
| 16.329 | 16.30 | 26.757 | 4.60 |
| 16.837 | 8.20 | 27.65 | 5.50 |
| 17.855 | 5.20 | 28.212 | 5.30 |
| 18.264 | 9.90 | | |

### Example 23 to Example 32: Preparation of Crystalline form II of Compound of Formula B

Referring to the preparation method in Example 22, the compound of formula B (20 mg) obtained in Preparation Example 1 was dissolved in different solvents to prepare 4-10 mg/mL solutions, and anti-solvents were slowly added at room temperature or 60 °C until a large amount of solid precipitated. The resulting solids were subjected to XRPD characterization, which showed the solids were the crystalline form II.

**Table 7. Preparation of crystalline form II and results**

| Exampl c | Sample concentration (mg/mL) | Solvent | Anti-solvent (Vₛₒₗᵥₑₙₜ:Vₐₙₜᵢ₋ₛₒₗᵥₑₙₜ) | Temperature (°C) | Result |
|---|---|---|---|---|---|
| 23 | 5 | Dichloromethane, 4 mL | *n*-Heptane (1:2.5) | Room temperature | Crystalli ne form II |
| 24 | 4 | Dichloromethane/methanol (V:V = 4:1), 5 mL | Methyl *tert*-butyl ether (1:2) | Room temperature | Crystalli ne form II |
| 25 | 10 | Dichloromethane/methanol (V:V = 4:1), 2 mL | Acetonitrile (1:4) | Room temperature | Crystalli ne form II |
| 26 | 5 | Dichloromethane/ethanol (V:V = 3:1), 4 mL | *n*-Heptane (1:3) | Room temperature | Crystalli ne form II |
| 27 | 5 | Dichloromethane/tetrahydrofuran (V:V = 3:1), 4 mL | *n*-Heptane (1:2) | Room temperature | Crystalli ne form II |
| 28 | 10 | Dichloromethane/methanol (V:V = 4:1), 2 mL | Methyl *tert*-butyl ether (1:3) | Room temperature | Crystalli ne form II |
| 29 | 10 | Dichloromethane/methanol (V:V = 1:4), 2 mL | *n*-Heptane (1:3) | Room temperature | Crystalli ne form II |
| 30 | 6.67 | Dimethylsulfoxide, 3 mL | Water (1:3) | 60 °C | Crystalli ne form II |
| 31 | 6.67 | Dimethylsulfoxide/methanol (V:V = 2:1), 3 mL | Water (1:3) | 60 °C | Crystalli ne form II |
| 32 | 6.67 | Dichloroethane, 3 mL | *n*-Heptane (1:3) | 60 °C | Crystalli ne form II |

### Example 33: Preparation of Crystalline form III of Compound of Formula B

The compound of formula B (20 mg) obtained in Preparation Example 1 was added to a reaction flask, and dichloromethane/methanol (V:V = 2:1, 3 mL) was added. The reaction liquid was stirred at room temperature for 30 min and then filtered. The filtrate was placed at room temperature, and the solvent was slowly volatilized to obtain the crystalline form III. The XRPD characterization pattern is substantially as shown in FIG. 5, and the characterization data are shown in Table 8.

**Table 8. XRPD characterization data on crystalline form III sample in Example 33**

| 2θ (°) | Relative peak intensity (%) | 2θ (°) | Relative peak intensity (%) |
|---|---|---|---|
| 4.447 | 100 | 18.458 | 2.00 |
| 10.367 | 5.1 | 20.464 | 2.3 |
| 13.437 | 4.1 | 24.324 | 2.1 |
| 13.955 | 1.8 | 25.259 | 4.00 |
| 18.141 | 2.70 | 25.845 | 6.60 |

### Comparative Example: Preparation of Other Crystalline forms of Compound of Formula B

### (1) Cooling crystallization method

Referring to the preparation method in Example 1, the crystalline forms of the compound of formula B were prepared by using the solvent systems in the table below. The results are as follows:

**Table 9. Preparation of other crystalline forms of compound of formula B by cooling crystallization method**

| Comparative Example | Solvent | Result |
|---|---|---|
| 1 | Dichloroethane/methanol (V:V = 2:1) | Clarified |
| 2 | N,N-Dimethylformamide/methanol (V:V = 2:1) | Clarified |

### (2) Slow evaporation crystallization

Referring to the preparation method in Example 33, the crystalline forms of the compound of formula B were prepared by using the solvent system in the table below. The results are as follows:

**Table 10. Preparation of other crystalline forms of compound of formula B by slow evaporation crystallization method**

| Comparative Example | Solvent | Result |
|---|---|---|
| 3 | Dichloroethane/methanol (V:V = 2:1) | Mixed crystal |

### Test Example 1: Competitive Suspension Experiments

Equal amounts (6 mg) of the crystalline form I sample of the compound of formula B obtained in Example 1, the crystalline form II sample of the compound of formula B obtained in Example 22, and the crystalline form III sample of the compound of formula B obtained in Example 33 were mixed. The resulting mixture was separately stirred at room temperature (25 °C) and at a high temperature (50 °C) for 24 h, 2 days, and 7 days in the following solvents, and the remaining solids were subjected to the XRPD test. The results are shown in the table:

**Table 11. Results of competitive suspension experiments**

| Solvent | Time | XRPD result (room temperature) | XRPD result (50 °C) |
|---|---|---|---|
| N,N-Dimethylformamide, 0.5 mL | 24 h | Crystalline form I + II | Crystalline form I + II |
| Dichloromethane/acetonitrile (V:V = 1:2), 0.5 mL | | Crystalline form I + II | Crystalline form I + II |
| Dichloromethane/ethanol (V:V = 1:4), 0.5 mL | | Crystalline form I + II | Crystalline form I + II |
| Water, 0.5 mL | 7 days | Crystalline form I + II | Crystalline form I + II |
| N,N-Dimethylformamide, 0.5 mL | | Crystalline form I | Crystalline form I |
| Dichloromethane/acetonitrile (V:V = 1:2), 0.5 mL | | Crystalline form I | Crystalline form I |
| Dichloromethane/ethanol (V:V = 1:4), 0.5 mL | | Crystalline form I | Crystalline form I |
| Water/acetone (V:V = 1:1), 1 mL | 2 days | Crystalline form I + II | Crystalline form I |
| Water/acetone (V:V = 2:8), 1 mL | | Crystalline form I + II | Crystalline form I |
| Water/acetone (V:V = 5:95), 1 mL | | Crystalline form I + II | Crystalline form I |

### Test Example 2: Solubility Test

The crystalline form I sample (5 mg) obtained in Example 1 and the crystalline form II sample (5 mg) obtained in Example 22 were added to a sample bottle, and 1 mL of SGF (simulated gastric fluid), FaSSIF (fasted state simulated intestinal fluid), FeSSIF (fed state simulated intestinal fluid), and water were separately added. The resulting suspensions were shaken at 37 °C at a rotation speed of 200 rpm for 24 h and filtered. The resulting filtrates were subjected to the concentration test, and the resulting solids were subjected to XRPD characterization. The results are shown in Table 13.

**Table 12. Preparation procedures for biological vehicles**

| Medium | Preparation procedure |
|---|---|
| SGF | Sodium chloride (200 mg) and concentrated hydrochloric acid (0.7 mL) were weighed into a 100 mL volumetric flask, water was added for dissolution and dilution, and the volume was brought to the mark. The pH value of the resulting solution was about 1.20. |
| FaSSIF | Sodium hydroxide (46.8 mg), sodium dihydrogen phosphate dihydrate (450 mg), and sodium chloride (609 mg) were weighed into a 100 mL volumetric flask, water was added for dissolution, and the volume was brought to the mark. The pH value of the resulting solution was about 6.50. |
| | SIF powder (44.8 mg) was weighed into a vial, and the FaSSIF buffer (20 mL) prepared above was added for dissolution. The resulting solution was stirred at room temperature for 2 h in the dark for later use. |
| FeSSIF | Sodium hydroxide (405 mg), glacial acetic acid (865.4 mg), and sodium chloride (1183 mg) were weighed into a 100 mL volumetric flask, water was added for dissolution, and the volume was brought to the mark. The pH value of the resulting solution was about 5.00. |
| | SIF powder (224 mg) was weighed into a vial, and the FeSSIF buffer (20 mL) prepared above was added for dissolution. The resulting solution was stirred at room temperature for 2 h in the dark for later use. |

**Table 13. Solubility test results for crystalline form I and crystalline form II**

| **Sample** | SGF | Water | FeSSIF | FaSSIF |
|---|---|---|---|---|
| | **Solubility (mg/mL)** | **Solubility (mg/mL)** | **Solubility (mg/mL)** | **Solubility (mg/mL)** |
| Crystalline form I | >5 | 0.0031 | 0.014 | 0.026 |
| Crystalline form II | >5 | 0.0015 | 0.0055 | 0.019 |

### Test Example 3: Solid Stability Study

The crystalline form I (5 mg) obtained in Example 1 and the crystalline form II (5 mg) obtained in Example 22 were weighed into sample bottles; the samples were subjected to solid-state stability studies after being placed under the conditions of 60 °C (closed) and 40 °C/75% RH (open); and sampling was performed on day 7 for XRPD characterization. The results are shown in Table 14.

**Table 14. Solid stability test results for crystalline form I and crystalline form II**

| **Sample** | **Time** | XRPD **result at 60 °C (closed)** | XRPD **result at 40 °C/75% RH (open)** |
|---|---|---|---|
| Crystalline form I | 7 days | Unchanged | Unchanged |
| Crystalline | 7 days | Unchanged | Unchanged |
| form II | | | |

### Test Example 4: Grinding Stability Study

The crystalline form I sample (3-5 mg) obtained in Example 1 was ground in a solvent, or the solid was directly ground for 5 min. The remaining solid was collected and subjected to XRPD characterization. The results are shown in Table 15.

**Table 15. Grinding stability test results for crystalline form I**

| **Method** | **Time** | **Solvent** | **Result** |
|---|---|---|---|
| Solid grinding | 2 min | / | The crystalline form was unchanged |
| | 5 min | / | The crystalline form was unchanged, but the crystallinity became worse |
| Solvent grinding | / | Acetone | The crystalline form was unchanged |
| | / | Acetonitrile | The crystalline form was unchanged |
| | / | Methanol | The crystalline form was unchanged |
| | / | Ethanol | The crystalline form was unchanged |
| | / | Dichloromethane | The crystalline form was unchanged |

### Test Example 5: In Vitro Efficacy Assay

### Assay drug: the compound of formula B obtained in Preparation Example 1

### 1. FGFR4 kinase activity inhibition assay

The FGFR4 protein kinase activity was determined by using the Caliper mobility shift assay technology. The compound was dissolved in DMSO and then diluted with a kinase buffer, and 5 µL of the compound (10% DMSO) at a 5-fold final reaction concentration was added to a 384-well plate. 10 µL of a 2.5-fold enzyme (FGFR4) solution was added, and then the mixture was incubated at room temperature for 10 min. 10 µL of a 2.5-fold substrate (FAM-labeled peptide and ATP) solution was added. The mixture was incubated at 28 °C for 30-60 min, and then 25 µL of a terminating solution was added to terminate the reaction. Conversion rate data were read on Caliper EZ Reader II (Caliper Life Sciences). The conversion rate was converted into inhibition data date (%inhibition rate = (max - sample conversion rate)/(max - min) × 100), wherein max referred to the conversion rate of the DMSO control, and min referred to the conversion rate of the enzyme-free control. The concentration and inhibition rate of the compound were used as the abscissa and ordinate to plot the curve, and the XLFit Excel Add-in version 4.3.1 software was used to fit the curve and calculate the IC₅₀ value.

The results show that the FGFR4 kinase activity inhibition (IC₅₀, nM) of the compound of formula B of the present disclosure was less than 5 nM.

### 2. Inhibition assay of compound on Huh-7 tumor cell proliferation

An Huh7 cell suspension was adjusted to 5 × 10e4 cells/mL or 2 × 10e4 cells/mL with DMEM + 2 mM Glutamine + 10% FBS medium. The cell suspension (100 µL) was added to each well of a 96-well cell culture plate at a final cell concentration of 5000 cells/well (72 h) or 2000 cells/well (168 h). The test compound was dissolved in DMSO as a 10 mmol stock solution. Compounds at 200× final concentration were prepared using the stock solution and DMSO, and 3× serial dilutions were prepared and diluted 20-fold with the medium. Finally, 10 µL of the corresponding 10-fold solution was added to each well of each cell strain, a single well for each drug concentration. The final treatment concentrations of the compounds were 3000 nM, 1000 nM, 333.3 nM, 111.1 nM, 37.04 nM, 12.35 nM, 4.12 nM, and 1.37 nM, with a final DMSO concentration of 0.5% per well. The cells were incubated in a 37 °C, 5% CO₂ incubator for 72 h or 168 h. After 72 h or 168 h of drug treatment, 100 µL of a CTG solution which was pre-melted and equilibrated to room temperature was added to each well according to CTG operation instructions, and the solution was mixed well using a microplate shaker for 2 min, left to stand at room temperature for 10 min, and measured for chemiluminescence signal values using an EnSpire plate reader. The cell survival rate was calculated using the following formula: (Vₛₐₘₚₗₑ - V_{blank})/(V_{vehicle control} - V_{blank}) × 100%, wherein Vₛₐₘₚₗₑ was the reading of the drug treatment group, Vvehicle control was the average value of the solvent control group, and V_{blank} was the average value of the blank control well. An S-type dose-survival rate curve was plotted using a non-linear regression model in the GraphPad Prism 5.0 software, and the IC₅₀ values were calculated.

The results show that the inhibition (IC₅₀, nM) of Huh7 tumor cell proliferation by the compound of formula B of the present disclosure was less than 10 nM.

### Test Example 6: Pharmacokinetic Study of Compound of Formula B in Rats

Assay drug: the compound of formula B obtained in Preparation Example 1.

Instrument: LC-MS/MS-13 (TQ5500, Triple quad), with the operation software of Xcalibur2.0.7 (Thermo Fisher Scientific, USA); LC-10ADvp liquid chromatography system (Shimadzu, Japan); Shimadzu LC-20AD liquid chromatography pump; Shimadzu CBM-20A system controller; Shimadzu CTO-20A column oven; and SIL-20AC autosampler (Shimadzu, Japan). Chromatographic column: Agilent Zorbax SB-CN 3.5u 100 × 2.1 mm; column temperature: 40 °C; mobile phase A: 0.1% aqueous formic acid solution; mobile phase B: a 0.1% solution of formic acid in acetonitrile; flow rate: 0.60 mL/min. The gradient elution was performed as follows: from 0 to 0.5 min, the mobile phase B was increased from 1% to 90%; from 0.5 to 1.2 min, 90% of the mobile phase B was maintained; starting from 1.21 min, the mobile phase B was changed back to 1%; and elution was stopped at 1.60 min.

Animals: 6 male SD rats were supplied by Shanghai Sippe-Bk Lab Animal Co., Ltd. The body weight range was 180-220 g. After purchase, the rats were bred in the laboratory of the experimental animal center for 2 days for later use. The rats were fasted 10-14 h before administration and 4 h after administration, and given ad libitum access to water during the assay. The rats were randomly divided into two groups, with 3 rats in each group. One group was intravenously injected, and the other group was intragastrically administered.

Preparation of intragastric administration solution: the compound of formula B (4.85 mg) was precisely weighed out, and a mixed aqueous solution (9.70 mL) containing 0.5% methylcellulose and 0.4% Tween-80 was added, followed by sonication at room temperature to prepare a 0.5 mg/mL suspension. Preparation of intravenous injection solution: the compound of formula B (2.79 mg) was precisely weighed out, and DMSO (21 µL), PEG200 (2.79 mL), and normal saline (4.164 mL) were added to prepare a 0.4 mg/mL solution.

Blood was collected in the intragastric administration group about 0.25 h before administration and 0.25 h, 0.5 h, 1 h, 1.5 h, 2 h, 4 h, 6 h, 8 h, 12 h, and 24 h after administration. Blood was collected in the intravenous administration group about 0.25 h before administration and 0.083 h, 0.25 h, 0.5 h, 0.75 h, 1 h, 2 h, 4 h, 8 h, 12 h, and 24 h after administration. The blood was collected from the jugular vein or other venipuncture at 0.25 mL/time point and anticoagulated with EDTA-K2 or EDTA-K3. The collected blood was placed on the ice. After the blood samples were collected, plasma was separated by centrifugation (centrifugation conditions: 6800 g/min, 6 min, 2-8 °C). The collected plasma samples were stored in a refrigerator at -80 °C for testing prior to analysis.

The compound of the formula B was accurately weighed out and formulated into different concentrations, and quantitative analysis was performed on a mass spectrometer to establish a standard curve. The concentration of the compound of formula B in the plasma described above was then measured to obtain the concentrations of the compound of formula B at different time points. All the measured data were collected and processed by relevant software, and pharmacokinetic parameters (mainly including kinetic parameters Tₘₐₓ, T_{1/2}, Cₘₐₓ, AUC₀₋ₜ, etc.) were calculated by adopting a statistical moment method.

Results: After the compound of formula B was intravenously injected at a dose of 2.0 mg/kg, the AUC₀₋ₜ was 3978.65 h*ng/mL, the half-life was 1.88 h, the volume distribution was 0.883 L/kg, and the clearance was 517.78 mL/h/kg; after the compound was intragastrically administered at 5 mg/kg, the AUC₀₋ₜ was 4835.41 h*ng/mL, the plasma concentration reached the peak value after 2.67 h, the Cₘₐₓ was 1218.83 ng/mL, the half-life was 1.95 h, and the absolute bioavailability was 48.61%.

### Test Example 7: Pharmacokinetic Study of Compound of Formula C in Rats

Assay drug: the compound of formula C obtained in Preparation Example 2.

The pharmacokinetics of the compound of formula C in rats was studied by using the same method as that in Test Example 6.

Results: After the compound of formula C was intravenously injected at a dose of 2.0 mg/kg, the AUC₀₋ₜ was 2553.74 h*ng/mL, the half-life was 1.23 h, the volume distribution was 0.973 L/kg, and the clearance was 803.23 mL/h/kg; after the compound was intragastrically administered at 5 mg/kg, the AUC₀₋ₜ was 2254.69 h*ng/mL, the plasma concentration reached the peak value after 2.5 h, the Cₘₐₓ was 596.53 ng/mL, the half-life was 1.24 h, and the absolute bioavailability was 35.32%.

In conclusion, the compound of formula B of the present disclosure has excellent FGFR4 kinase inhibitory activity and Huh-7 tumor cell proliferation inhibitory activity, the pharmacokinetic effect is better compared to an enantiomer or a racemate thereof, and the crystalline form, particularly the preferred crystalline form, has good solubility, stable quality, stable thermodynamics, and desirable druggability.

## Claims

1. A compound of formula B in a crystalline form,

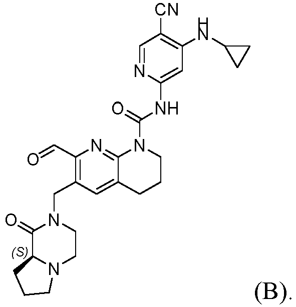

2. The compound of formula B in the crystalline form according to claim 1, being an anhydrate.

3. The compound of formula B in the crystalline form according to claim 1 or 2, being crystalline form I, wherein the crystalline form I
(1) has characteristic peaks at 2θ angles of 7.4±0.2° and 25.0±0.2° by X-ray powder diffraction using Cu-Kα radiation, or
(2) has characteristic peaks at 2θ angles of 7.4±0.2°, 15.9±0.2°, 17.4±0.2°, 20.9±0.2°, and 25.0±0.2° by X-ray powder diffraction using Cu-Kα radiation, or
(3) has characteristic peaks at 2θ angles of 7.4±0.2°, 9.8±0.2°, 15.9±0.2°, 17.4±0.2°, 20.9±0.2°, and 25.0±0.2° by X-ray powder diffraction using Cu-Kα radiation, or
(4) has characteristic peaks at 2θ angles of 7.4±0.2°, 9.8±0.2°, 15.9±0.2°, 17.4±0.2°, 20.9±0.2°, 22.4±0.2°, and 25.0±0.2° by X-ray powder diffraction using Cu-Kα radiation, or
(5) has characteristic peaks at 2θ angles of 7.4±0.2°, 9.8±0.2°, 11.7±0.2°, 12.0±0.2°, 14.6±0.2°, 15.9±0.2°, 17.4±0.2°, 19.7±0.2°, 20.9±0.2°, 22.4±0.2°, 23.6±0.2°, and 25.0±0.2° by X-ray powder diffraction using Cu-Kα radiation, or
(6) has characteristic peaks at 2θ angles of 7.4±0.2°, 9.8±0.2°, 11.7±0.2°, 12.0±0.2°, 14.6±0.2°, 15.5±0.2°, 15.9±0.2°, 17.4±0.2°, 18.8±0.2°, 19.7±0.2°, 20.9±0.2°, 22.0±0.2°, 22.4±0.2°, 23.6±0.2°, 25.0±0.2°, and 27.8±0.2° by X-ray powder diffraction using Cu-Kα radiation, or
has an X-ray powder diffraction pattern substantially as shown in FIG. 1 or FIG. 3 by using Cu-Kα radiation.

4. The compound of formula B in the crystalline form according to claim 1 or 2, being crystalline form I, wherein a single crystal of the crystalline form I is examined using CuKα radiation and belongs to a monoclinic crystal system with a *P*2₁ space group, and unit cell parameters are as follows: a = 10.8374(2) Ǻ, b = 23.6358(3) Ǻ, c = 10.96160(10) Ǻ, α = 90°, β = 117.360(2)°, γ = 90°, V = 2493.73(7) Ǻ³.

5. The compound of formula B in the crystalline form according to claim 1 or 2, being crystalline form II, wherein the crystalline form II
(1) has characteristic peaks at 2θ angles of 7.6±0.2° and 14.0±0.2° by X-ray powder diffraction using Cu-Kα radiation, or
(2) has characteristic peaks at 2θ angles of 7.6±0.2°, 14.0±0.2°, 15.2±0.2°, 20.4±0.2°, and 24.8±0.2° by X-ray powder diffraction using Cu-Kα radiation, or
(3) has characteristic peaks at 2θ angles of 7.6±0.2°, 9.5±0.2°, 14.0±0.2°, 15.2±0.2°, 16.3±0.2°, 20.4±0.2°, 21.6±0.2°, 21.9±0.2°, 23.2±0.2°, 23.5±0.2°, and 24.8±0.2° by X-ray powder diffraction using Cu-Kα radiation, or
(4) has characteristic peaks at 2θ angles of 7.6±0.2°, 9.5±0.2°, 10.2±0.2°, 12.7±0.2°, 14.0±0.2°, 15.2±0.2°, 16.3±0.2°, 16.8±0.2°, 18.3±0.2°, 20.4±0.2°, 21.6±0.2°, 21.9±0.2°, 23.2±0.2°, 23.5±0.2°, and 24.8±0.2° by X-ray powder diffraction using Cu-Kα radiation, or
(5) has characteristic peaks at 2θ angles of 7.6±0.2°, 9.5±0.2°, 10.2±0.2°, 12.7±0.2°, 14.0±0.2°, 15.2±0.2°, 16.3±0.2°, 16.8±0.2°, 17.9±0.2°, 18.3±0.2°, 20.4±0.2°, 21.6±0.2°, 21.9±0.2°, 23.2±0.2°, 23.5±0.2°, 24.8±0.2°, 26.8±0.2°, 27.7±0.2°, and 28.2±0.2° by X-ray powder diffraction using Cu-Kα radiation, or
has an X-ray powder diffraction pattern substantially as shown in FIG. 4 by using Cu-Kα radiation.

6. The compound of formula B in the crystalline form according to claim 1 or 2, being crystalline form III, wherein the crystalline form III
(1) has characteristic peaks at a 2θ angle of 4.4±0.2° by X-ray powder diffraction using Cu-Kα radiation, or
(2) has characteristic peaks at 2θ angles of 4.4±0.2°, 10.4±0.2°, 13.4±0.2°, 25.3±0.2°, and 25.8±0.2° by X-ray powder diffraction using Cu-Kα radiation, or
(3) has characteristic peaks at 2θ angles of 4.4±0.2°, 10.4±0.2°, 13.4±0.2°, 14.0±0.2°, 18.1±0.2°, 18.5±0.2°, 20.5±0.2°, 24.3±0.2°, 25.3±0.2°, and 25.8±0.2° by X-ray powder diffraction using Cu-Kα radiation, or
has an X-ray powder diffraction pattern substantially as shown in FIG. 5 by using Cu-Kα radiation.

7. A pharmaceutical composition, comprising the compound of formula B in the crystalline form or one or a mixture of more of the crystalline form I, the crystalline form II, and the crystalline form III of the compound of formula B according to any one of claims 1-6, and optionally one or more pharmaceutically acceptable carriers.

8. Use of the compound of formula B in the crystalline form according to any one of claims 1-6 or the pharmaceutical composition according to claim 7 in the manufacture of a medicament for treating a disease associated with FGFR4 activity or expression.

9. The use according to claim 8, wherein the disease associated with the FGFR4 activity or expression is selected from cancers, e.g., lung cancer, bladder cancer, breast cancer, gastric cancer, liver cancer, salivary gland sarcoma, ovarian cancer, prostate cancer, cervical cancer, epithelial cell carcinoma, multiple myeloma, pancreatic cancer, lymphoma, chronic myelogenous leukemia, lymphocytic leukemia, and cutaneous T-cell lymphoma; preferably, the lung cancer is non-small cell lung cancer; or
the disease associated with the FGFR4 activity or expression is selected from bone-related diseases, e.g., osteogenesis imperfecta, achondroplasia, dwarfism, and Crouzon syndrome; or
the disease associated with the FGFR4 activity or expression is selected from T cell-regulated inflammation and autoimmune diseases, e.g., rheumatoid arthritis, collagen II arthritis, multiple sclerosis, systemic lupus erythematosus, psoriasis, juvenile onset diabetes, Sjogren's syndrome, thyroid diseases, sarcoidosis, inflammatory bowel disease, and celiac disease.
